## Europäisches Patentamt
## European Patent Office
## Office européen des brevets

(19)

(11) Veröffentlichungsnummer: **0 010 672**
**B1**

(12) # EUROPÄISCHE PATENTSCHRIFT

(45) Veröffentlichungstag der Patentschrift:
**02.09.81**

(51) Int. Cl.³: **C 07 C 21/18**, C 07 C 17/34

(21) Anmeldenummer: **79103964.7**

(22) Anmeldetag: **15.10.79**

(54) **Verfahren zur Herstellung von 1-Fluor-1,2-dichloräthylen durch Dehydrochlorierung von 1-Fluor-1,1,2-trichloräthan.**

(30) Priorität: **27.10.78 DE 2846810**

(43) Veröffentlichungstag der Anmeldung:
**14.05.80 Patentblatt 80/10**

(45) Bekanntmachung des Hinweises auf die Patenterteilung:
**02.09.81 Patentblatt 81/35**

(84) Benannte Vertragsstaaten:
**DE FR GB IT NL**

(56) Entgegenhaltungen:
**DE-C- 816 698**
**DE-C- 964 043**
**GB-A- 813 175**
**US-A- 2 877 277**

(73) Patentinhaber: **BAYER AG, Zentralbereich Patente, Marken und Lizenzen, D-5090 Leverkusen 1, Bayerwerk (DE)**

(72) Erfinder: **Mitschke, Karl-Heinz, Dr., Am Berg 22, D-5068 Odenthal (DE)**
Erfinder: **Niederprüm, Hans, Dr., Hofstrasse 27, D-4019 Monheim (DE)**

### Verfahren zur Herstellung von 1-Fluor-1,2-dichloräthylen durch Dehydrochlorierung von 1-Fluor-1,1,2-trichloräthan

Die vorliegende Erfindung betrifft ein verbessertes Verfahren zur Herstellung von 1-Fluor-1,2-dichloräthylen.

1-Fluor-1,2-dichloräthylen, eine an sich seit langem bekannte Substanz, ist vielseitig einsetzbar; so zum Beispiel als Zwischenprodukt für organische Synthesen, als Lösungsmittel oder als Treibmittel für Kunststoffe.

Die Herstellung von 1-Fluor-1,2-dichloräthylen erfolgte bisher durch relativ aufwendige Verfahren. So ist es zum Beispiel bekannt, 1-Fluor-1,2-dichloräthylen durch Umsetzung von 1-Fluor-1,1,2,2-tetrachloräthan mit Zink in organischen Lösungsmitteln herzustellen (vgl. z. B. Am. Soc. 58, 402 [1936]). Dieses Verfahren ist sowohl von der Herstellung des Ausgangsmaterials als auch von der Aufarbeitung der entstehenden Zinkhalogenide sehr aufwendig, so daß es für eine technische Durchführung praktisch nicht in Frage kommt. 1-Fluor-1,2-dichloräthylen kann auch durch thermische Abspaltung von Halogenwasserstoff aus 1-Fluor-1,1,2-trichloräthan bei ca. 550°C in Gegenwart von Raschigringen erhalten werden (vgl. z. B. US-PS 26 28 989 oder Ind. Eng. Chem. 43, 1253 [1951]). Die bei diesem Verfahren notwendigen hohen Temperaturen sind im Zusammenhang mit dem bei der Reaktion freiwerdenden Chlorwasserstoff großtechnischen Anlagen problemhaft. Ferner haben solche Pyrolysereaktionen den Nachteil, daß eine Reihe unerwünschter Nebenprodukte entsteht.

Ferner ist bekannt, daß Dehydrohalogenierungsreaktionen von Chlor-Fluor-Äthanen mit wäßrigen Basen in Gegenwart von organischen Lösungsmitteln oder Emulgatoren stattfinden. Diese Verfahren führen bei Ausgangsmaterialien mit relativ niedrigen Siedepunkten zu unbefriedigenden Ausbeuten oder Nebenproduktbildung (vgl. Houben—Weyl, Methoden der organischen Chemie, Band 5, Kapitel 3, Seite 388; US-PS 2 709 181).

Ein weiteres Verfahren zur Herstellung von Fluorchloräthylenen wird in DE-C 816 698 beschrieben. Nach diesem Verfahren erhöht man jedoch nur geringe Ausbeuten, unter Druck können sogar nur Spuren von 1-Fluor-1,2-dichloräthylen erhalten werden. Eine Nicht-Abtrennung des Reaktionsproduktes aus dem Reaktionsgemisch hat offenbar zur Folge, daß Zersetzungsreaktionen eintreten und somit eine befriedigende Ausbeute nicht erzielt werden kann.

Aufgabe der vorliegenden Erfindung war es, ein einfaches und wirtschaftliches Verfahren zur Herstellung von 1-Fluor-1,2-dichloräthylen bereitzustellen.

Gegenstand der vorliegenden Erfindung ist nun ein Verfahren zur Herstellung von 1-Fluor-1,2-dichloräthylen durch Dehydrohalogenierung von 1-Fluor-1,1,2,-trichloräthan, das dadurch gekennzeichnet ist, daß 1-Fluor-1,1,2-trichloräthan mit basisch reagierenden Substanzen in Gegenwart von Wasser bei erhöhter Temperatur und unter Druck umgesetzt wird, wobei das gebildete 1-Fluor-1,2-dichloräthan fortwährend aus dem Reaktionsgemisch entfernt wird.

Überraschenderweise hat es sich herausgestellt, daß die Dehydrohalogenierung von Fluor-Chlor-Alkanen auch ohne Zusätze von organischen Lösungsmitteln oder Emulgatoren (vgl. Houben—Weyl, Methoden der organischen Chemie, Band 5, Kapitel 4, Seite 741 und Band 5, Kapitel 3, Seite 388; US-PS 2 709 181) in kurzer Zeit und hohen Ausbeuten gelingt, wenn die Umsetzung von 1-Fluor-1,1,2-trichloräthan und den basisch reagierenden Stoffen in Gegenwart von Wasser in einer Druckreaktion bei erhöhter Temperatur durchgeführt wird. Die erfindungsgemäße, in der flüssigen Phase stattfindende Reaktion zeigt den Vorteil, daß keinerlei Emulgatoren benötigt werden, daß ohne organische Lösungsmittel gearbeitet werden kann und somit die Bildung unerwünschter Nebenprodukte vermieden wird, daß die Aufarbeitung relativ problemlos ist, und daß eine sehr gute Raum-Zeit-Ausbeute erzielt wird.

Das erfindungsgemäße Verfahren wird in einer allgemeinen Ausführung so durchgeführt, daß in einem Druckbehälter, der mit den üblichen Zusätzen, wie z. B. Rührer, gegebenenfalls Druckkühler, Druckkonstanthalteventile usw. versehen ist, eine der Reaktionskomponenten vorgelegt und die andere, vorzugsweise der basisch wirkende Stoff, in Wasser gelöst oder suspendiert, zudosiert wird, nachdem der Autoklav zuvor vorzugsweise mit einem Inertgas, wie beispielsweise Stickstoff, unter einen Vordruck gesetzt wird. Das während der Reaktion entstehende 1-Fluor-1,2-dichloräthylen wird kontinuierlich über das Druckkonstanthalteventil in gekühlte Behälter kondensiert und nach dem Trocknen (z. B. mit Natriumsulfat) destilliert. Eventuell im Sumpf verbleibendes Ausgangsmaterial kann gegebenenfalls erneut zur Reaktion gebracht werden.

Die erfindungsgemäße Umsetzung erfolgt bei Temperaturen von 30 bis 150°C, vorzugsweise im Bereich von etwa 60 bis 130°C und bei Drücken von 0,5 bis 10 bar, vorzugsweise von 1 bis 6 bar. Unter den beschriebenen Bedingungen setzt die Reaktion momentan ein und ist nach relativ kurzer Zeit beendet (abhängig von Ansatzgröße, Druck- und Temperatur-Verhältnissen). Im allgemeinen ist eine Reaktionszeit bis 3 Stunden ausreichend. Zu langes Erhitzen führt sogar zu unerwünschten Nebenprodukten. Das erfindungsgemäße Verfahren kann sowohl kontinuierlich — auch mehrstufig — als auch diskontinuierlich durchgeführt werden.

Als Dehydrohalogenierungsmittel eignen sich basisch reagierende anorganische und/oder organische Substanzen, wie z. B. Alkalioxide oder -Hydroxide, wie Natrium- oder Kaliumoxide oder -Hydroxide, Erdalkalioxide oder Erdalkalihydroxide, Alkalicarbonate oder Alkaliphosphate oder Alkalicarboxylate, Ammoniak und Amine, wie z. B. Trialkyl-, Dialkyl- oder Monoalkylamine. Bevorzugt wird Natriumhydroxid eingesetzt. Es ist ohne weiteres möglich, auch Gemische von basisch reagierenden Substanzen einzusetzen, wie z. B. Natriumhydroxid und Amine, wobei es besonders vorteilhaft ist, die Amine nur in katalytischen Mengen einzusetzen, z. B. etwa 0,01 bis 0,2, vorzugsweise 0,05 bis 0,1 Mole pro Mol 1-Fluor-1,1,2-trichloräthan.

Für die Umsetzungen werden etwa äquimolare Mengen an basisch reagierenden Substanzen eingesetzt, in der Regel 0,9—2 Mol, vorzugsweise 1—1,4 Mol pro Mol eingesetztes 1-Fluor-1,1,2-trichloräthan.

Das als Ausgangsmaterial eingesetzte 1-Fluor-1,1,2-trichloräthan kann in technischer Qualität — wie es aus der Herstellung durch Fluorierung von Trichloräthylen erhalten wird — eingesetzt werden. Besondere Reinigungsschritte sind nicht erforderlich.

Wasser muß in einer solchen Menge zugeführt werden, daß der basisch wirkende Stoff gelöst bzw. zumindest suspendiert werden kann.

Das erfindungsgemäße Verfahren soll nun anhand der folgenden Beispiele noch näher erläutert werden. Prozentangaben beziehen sich soweit nicht anders angegeben auf Gewichtsprozente. Die angegebenen Druckwerte beziehen sich auf Überdruck.

### Beispiel 1

Ein Autoklav, versehen mit Rührer, beheizbarem Druckkühler und Druckkonstanthalteventil, wurde mit 303 g (2 Mol) 1-Fluor-1,1,2-trichloräthan und 100 g Wasser gefüllt, mit Stickstoff unter einen Druck von ca. 4 bar gesetzt und danach auf eine Temperatur von ca. 100°C gebracht. Danach wurde eine wäßrige Lösung von 96 g (2,4 Mol) Natriumhydroxid in 320 g Wasser zugepumpt. Das während ca. 0,75 Stunden sich bildende Olefin wurde in einem auf ca. −10°C gekühlten Behälter kondensiert. Anschließend wurde das Kondensat über Natriumsulfat getrocknet und destilliert. Es wurden 210 g (91% der Theorie) 1-Fluor-1,2-dichloräthylen (Kp: 35—27°C) erhalten. Die Verbindung wurde $^1$H- und $^{19}$F-NMR- sowie IR-spektroskopisch und gaschromatographisch identifiziert.

### Beispiel 2

In einen Autoklaven wurden 227 g (1,5 Mol) 1-Fluor-1,1,2-trichloräthan und 185,5 g (1,75 Mol) Natriumcarbonat und 600 ml Wasser gefüllt und die Mischung nach dem Aufdrücken von ca. 3 bar Stickstoff unter Rühren auf ca. 100°C erhitzt. Nach ca. 1 Stunde wurde aufgearbeitet. Es wurden 142 g (82% der Theorie) an 1-Fluor-1,2-dichloräthylen erhalten.

### Beispiel 3

Entsprechend Beispiel 1 wurde zu einer Mischung aus 151 g (1 Mol) 1-Fluor-1,1,2-trichloräthan, 10 g (0,1 Mol) Triäthylamin und 50 ml Wasser, die auf ca. 100°C und einen Druck von 5 bar gebracht wurden, eine Lösung von 40 g (1 Mol) Natriumhydroxid in 150 g Wasser dosiert. Nach ca. 0,75 Stunden wurde aufgearbeitet. Es wurden 102 g (89% der Theorie) an 1-Fluor-1,2-dichloräthylen erhalten.

**Patentansprüche**

1. Verfahren zur Herstellung von 1-Fluor-1,2-dichloräthylen durch Dehydrohalogenierung von 1-Fluor-1,1,2-trichloräthan, dadurch gekennzeichnet, daß 1-Fluor-1,1,2-trichloräthan mit basisch reagierenden Substanzen in Gegenwart von Wasser bei erhöhter Temperatur und unter Druck umgesetzt wird, wobei das gebildete 1-Fluor-1,2-dichloräthan fortwährend aus dem Reaktionsgemisch entfernt wird.

2. Verfahren nach Anspruch 1, dadurch gekennzeichnet, daß die Dehydrohalogenierung mit Natriumhydroxid, gegebenenfalls im Gemisch mit katalytischen Mengen an Aminen, als basisch reagierende Substanz, durchgeführt wird.

3. Verfahren nach Anspruch 1 oder 2, dadurch gekennzeichnet, daß die Dehydrohalogenierung bei Temperaturen von 30 bis 150°C und bei einem Druck von 0,5 bis 10 bar durchgeführt wird.

**Claims**

1. Process for the production of 1-fluoro-1,2-dichloroethylene by dehydrohalogenating 1-fluoro-1,1,2-trichloroethane, characterised in that 1-fluoro-1,1,2-trichloroethane is reacted with basically reacting substances under pressure at elevated temperature in the presence of water, the 1-fluoro-1,2-dichloroethane formed being continuously removed from the reaction mixture.

2. Process according to Claim 1, characterised in that the dehydrohalogenation is carried out with sodium hydroxide, optionally in admixture with catalytic amounts of amines, as the basically reacting substance.

3. Process according to Claim 1 or 2, characterised in that the dehydrohalogenation is conducted at temperatures of 30 to 150°C and under a pressure of 0.5 to 10 bars.

**Revendications**

1. Procédé de production de 1-fluoro-1,2-dichloréthylène par déshydrohalogénation de 1-fluoro-1,1,2-trichloréthane, caractérisé en ce qu'on fait réagir du 1-fluoro-1,1,2-trichloréthane avec des substances à réaction basique en présence d'eau à une température élevée et sous pression, le 1-fluoro-1,2-dichloréthane formé étant éliminé du mélange réactionnel en continu.

2. Procédé suivant la revendication 1, caractérisé en ce que la déshydrohalogénation est effectuée en utilisant comme substance à réaction basique l'hydroxyde de sodium, éventuellement en mélange avec des quantités catalytiques d'amines.

3. Procédé suivant la revendication 1 ou 2, caractérisé en ce que la déshydrohalogénation est conduite à des températures de 30 à 150°C et à une pression de 0,5 à 10 bars.